# EUROPEAN PATENT SPECIFICATION

(11) **EP 1 473 300 B1**
(45) Date of publication and mention of the grant of the patent: **26.11.2008**
(21) Application number: 02796785.0
(22) Date of filing: 11.12.2002
(51) Int. Cl.: C07J 7/00, A61K 31/57, A61K 31/573, A61P 17/06

(54) **PHARMACEUTICAL COMPOSITION FOR THE TREATMENT OF PSORIASIS AND OTHER SKIN DISEASES**
PHARMAZEUTISCHE ZUSAMMENSETZUNG ZUR BEHANDLUNG VON PSORIASIS UND ANDEREN HAUTKRANKHEITEN
COMPOSITION PHARMACEUTIQUE DESTINEE AU TRAITEMENT DU PSORIASIS ET D'AUTRES PATHOLOGIES CUTANEES

(30) Priority: 12.12.2001 ES 200102836
(43) Date of publication of application: 03.11.2004
(73) Proprietor: Litaphar Laboratorios S.A., 20730 Azpeitia (Guipuzcoa) (ES)
(72) Inventor: PIDAL FERNANDEZ, Rosalia, E-08036 Barcelona (ES); TOMASI SCIANO, Giuseppe, I-98076 S. Agata Militello-(Messina) (IT); LARRANAGA LAZCANO, Lidia, E-20730 Azpeitia-(Guipuzcoa) (ES)
(74) Representative: Barlocci, Anna
(86) International application number: PCT/ES2002/000591
(87) International publication number: WO 2003/053991

(56) References cited:
- WO-A-98/36753
- US-A- 5 972 920
- SWINKELS O.Q.J. ET AL.: 'The influence of a topical corticosteroid on short-contact high-dose dithranol therapy' BRITISH J. OF DERMATOLOGY vol. 145, 2001, pages 63 - 69, XP002978891
- BARAN R. ET AL.: 'Topical treatment of nail psoriasis with a new corticoid-containing nail lacquer formulation' J. OF DERMATOLOGICAL TREATMENT vol. 10, 1999, pages 201 - 204, XP002978895

## Description

The invention is within the field of the therapy and prophylaxis of psoriasis and other inflammatory or allergic skin diseases, by the means of the topical application of corticosteroids.

### BACKGROUND ART

Inflammatory or allergic skin diseases, especially the proliferative ones, have always been a source of physical and psychological problems for humans and other animals. For some of these skin diseases, such as psoriasis, there is no cure. Psoriasis is a chronic and recurring disease recognized by its silvery scaled eruptions and plaques of various sizes. The scaling is caused by an increase and an abnormally high production of cutaneous cells. The cause of this accelerated cellular growth is unknown, but it is believed that immunological mechanisms may play an important role. The disease is common, affecting from 2 to 4% of the Caucasian population. There are very distinct degrees of psoriasis, regarding the intensity of the cutaneous disorder and the extent and location of the affected areas.

No known therapeutic method can cure psoriasis, but the majority of cases can be controlled. In the less severe cases of the disease, treatment with pomades or emollient creams that keep the skin hydrated can be sufficient. In the more severe cases, the antineoplastic methotrexate or cyclosporin, both of which provoke serious side effects, can be used. In many of the moderate cases of psoriasis, however, topical formulations (pomades, creams, gels, lotions...) containing corticosteroids are used by applying them underneath an occlusive covering made of cellophane or polyethylene, or incorporating them into an adhesive bandage. Depending on the affected area, applying these topical formulations can represent a real practical problem for the patient, especially during the day.

In addition to the problems inherent in their topical application, another problem with corticosteroids is that the disease does not always respond to treatment and, when it does, it tends to relapse rapidly. The frequency of relapses is essential in the treatment of psoriasis because the prolonged application of topical corticosteroids causes local side effects (atrophic alterations, loss of collagen, stretch marks, hypertrichosis, telangiectasia, pigmentary disorders) and loss of efficacy (tachyphylaxis). Therefore, if the relapses are far apart from each other -for example, many months or several years apart- the patient thinks that they have been cured.

For the treatment of skin diseases, a large number of topical corticosteroids are used which, in Europe, are classified into four groups according to their potency: weak, intermediate, high or very high. One of the very highly potent topical corticosteroids used for the treatment of skin diseases is 17-clobetasol propionate (CAS RN = 25122-46-7), sold in the United States in the form of a cream, pomade (unguent), gel or solution. In Spain, 17-clobetasol propionate creams and pomades have been approved for the following indications: treatment of inflammatory or allergic skin diseases such as psoriasis, contact dermatitis, atopic dermatitis, seborrheic dermatitis, insect bites, inflammatory dermatoses, eczema, granuloma annulare, lichen planus, discoid lupus erythematosus, localized neurodermatitis, pruritus ani, xerosis, allergic otitis externa, pemphigus, polymorphic light eruption and minor burns.

Some combinations of 17-clobetasol propionate with other active ingredients have been described for the treatment of psoriasis and other skin diseases. Thus, for example, in the patent US 5.972.920, the use of liquid formulations of 17-clobetasol propionate with zinc pyrithione is described. Patent application WO 98/36753 claims the combination of 17-clobetasol propionate with tazarotene, an antipsoriatic from the retinoid class. But betamethasone valerate, and not 17-clobetasol propionate, is the only highly or very highly potent corticosteroid whose combination with tazarotene is illustrated in this document. In the patent US 5.874.074 an occlusive lotion including a soluble film-forming polymer for the application of corticosteroids is described.

### SUMMARY OF THE INVENTION

In one of its aspects, the present invention provides pharmaceutical compositions for topical administration that comprise a therapeutically effective amount of 17-clobetasol propionate, adequate amounts of excipients acceptable for pharmaceutical or cosmetic formulations for topical administration, and a therapeutically effective amount of a hydroxy derivative of progesterone having formula (I), or of one of its pharmaceutically acceptable esters, wherein, independently from each other, R6 is selected from hydrogen or methyl; R11 is selected from hydrogen or hydroxyl; and R17 is selected from hydrogen or hydroxyl, with the proviso that R11 and R17 are not hydrogen simultaneously.

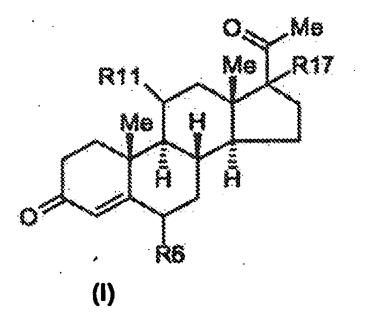

The products (I) include various stereoisomers, that stem from variations in the carbons, the stereochemistry of which is not indicated in the formula. The present invention refers to any of the stereoisomers and mixtures of the same. Also included in the present invention are the pharmaceutically acceptable solvates, particularly hydrates.

Pharmaceutically acceptable esters are understood as those coming from the esterification of the hydroxyl groups of (I) with pharmaceutically acceptable carboxylic acids, preferably those with a linear chain of between two and six carbon atoms, with acetic acid being particularly preferred.

In one particular embodiment, the compositions comprise the product of formula (I) wherein R6 is hydrogen, R11 is hydroxyl and R17 is hydrogen, with compositions comprising 11-α-hydroxyprogesterone or its acetate being especially preferred. In another particular embodiment, the compositions comprise the product of formula (I) wherein R6 is hydrogen, R11 is hydrogen and R17 is hydroxyl, with compositions comprising 17-α-hydroxyprogesterone, its acetate or its hexanoate being especially preferred. In another particular embodiment, the compositions comprise the product of formula (I) wherein R6 is methyl, R11 is hydrogen and R17 is hydroxyl, with compositions comprising medroxyprogesterone or its acetate being especially preferred.

Although the composition of the present invention can be applied in various galenic topical forms (creams, pomades, gels, solutions, etc.), its application in the form of a lotion is especially preferred. Therefore, in a preferred embodiment, the topical compositions of the present invention are lotions that have one or more pharmaceutically acceptable monoalcohols as a dissolvent, and one or more pharmaceutically acceptable polyols as co-dissolvents at a proportion between 20% and 50%, in addition to water at a proportion between 10% and 30%. Compositions in which the monoalcohol dissolvent is selected from ethanol, isopropanol and its combinations, and the polyol co-dissolvent is selected from propylene glycol, glycerin, sorbitol and its combinations, are even more preferable. In addition, the lotion can contain small quantities of other excipients such as stabilizers (e.g. ascorbic acid), preservatives (e.g. bencylic alcohol or (C1-C4)-alkyl parabenes), perfumes, etc. The lotion can be applied directly or with a spray, on parts of the body where its excipients would not cause irritation (not on mucous membranes). The lotion can also be incorporated into a covering made of flexible and absorbent material, ideal for applying to the skin. One very convenient means of application is simply by anointing the affected areas of the skin with the lotion on a cotton ball and then leaving it to dry.

The preferred compositions are those in which the amount of 17-clobetasol propionate is between 0.05% and 0.4% in weight, and the amount of hydroxy derivative of progesterone having formula (I) or its pharmaceutically acceptable ester is between 0.1% and 1.5%. Even more preferred are those compositions in which these quantities are between 0.1% and 0.2%, and between 0.3% and 1%, respectively.

The composition that is the subject of the present invention can be used for therapeutic or cosmetic purposes, in various diseases or cutaneous disorders. Nonetheless, the composition is especially useful for the prophylactic or curative treatment of inflammatory or allergic skin diseases such as psoriasis, contact dermatitis, atopic dermatitis, seborrheic dermatitis, insect bites, inflammatory dermatoses, eczema, granuloma annulare, lichen planus, discoid lupus erythematosus, localized neurodermatitis, pruritus ani, xerosis, allergic otitis externa, pemphigus, polymorphic light eruption and minor burns. Therefore, another aspect of the present invention is the use of a combination of 17-clobetasol propionate with a hydroxy derivative of progesterone having formula (I) as defined previously, or with one of its pharmaceutically acceptable esters, for the preparation of a pharmaceutical composition for topical administration for the treatment of inflammatory or allergic skin diseases, especially for the treatment of psoriasis, the treatment of which is explained in the attached example. For the treatment of psoriasis, it is suitable to apply the lotion of the present invention simultaneously with an emollient cream typically used for skin conditions.

One of the advantages of the composition of the present invention with respect to known treatments with topical corticosteroids (e.g. 17-clobetasol propionate alone) is that relapses are very spread apart. Another advantage is that many of the local side effects usually associated with the topical application of corticosteroids (atrophic alterations, loss of collagen, stretch marks, hypertrichosis, telangiectasia, pigmentary disorders) are avoided. Apparently, the hydroxy derivative of progesterone having formula (I) and its esters have a regenerating effect on the skin that makes up for the erosion caused by the corticosteroid treatment. Furthermore, compared to the 17-clobetasol propionate creams or pomades, the lotion of the present invention has the advantage that its application is more pleasant for the patient, as it does not smear nor stain, it evaporates rapidly and does not need an occlusive covering.

Throughout the description and the claims the word "comprise" and its variants do not intend to exclude other technical characteristics, additives, components or steps.

Additional objects, advantages and features of the invention will become apparent to those skilled in the art upon examination of the description or may be learned by practice of the invention. The following examples and drawings are provided by way of illustration, and is not intended to be limiting of the present invention.

### DETAILED DESCRIPTION OF A PARTICULAR EMBODIMENT

With the following composition in weight:
- 17-Clobetasol propionate 0.15%
- 11-α-Hydroxyprogesterone 0.75%
- Glycerin 5%
- Propylene glycol 35%
- Distilled water 20%
- Ethanol 96% (to fill up)
a lotion was prepared by the following process: in a first container in bain-marie the 17-clobetasol propionate, 11-α-hydroxyprogesterone, propylene glycol and most of the ethanol were dissolved. In a second container in bain-marie the glycerin was dissolved in water. The contents of the second container were added to the first slowly and stirred. Finally, it was filled up with ethanol to the desired volume. -

The applicability of the above lotion has been studied in a group of twelve Caucasian patients (seven women and five men), of ages ranging from 31 to 45 years. All patients had stable psoriatic plaques, more or less severe, on elbows, knees, buttocks or gemellus. All patients had been in maintenance treatment for the disease and, in half of the cases, this treatment included the application of 17-clobetasol propionate at some point.

After the reappearance of the plaques, each patient applied the above-described lotion on the plaques once a day by dabbing them with a cotton swab impregnated with the lotion. Treatment was accompanied by the application of a moisturizing cream that was applied two times a day, once after applying the lotion and then again twelve hours later. After three days, improvement was seen in more than half of the patients, and after a week, the plaques had practically disappeared in ten out of the twelve cases; at this point application of the lotion was then ceased and treatment with only the moisturizing cream continued. In addition, according to the patients, after treatment their skin had a much better appearance than it did with previous treatments. In nine of the study patients there was no relapse during a one-year follow-up after treatment with the lotion, whereas the disease previously reappeared in these patients in less than six months.

## Claims

1. A pharmaceutical composition for topical administration that comprises a therapeutically effective amount of 17-clobetasol propionate, adequate amounts of excipients acceptable for pharmaceutical or cosmetic formulations for topical administration, and a therapeutically effective amount of a hydroxy derivative of progesterone having formula (I), or of one of its pharmaceutically acceptable esters, wherein, independently from each other, R6 is selected from hydrogen or methyl, R11 is selected from hydrogen or hydroxyl; and R17 is selected from hydrogen or hydroxyl, with the proviso that R11 and R17 are not hydrogen simultaneously.

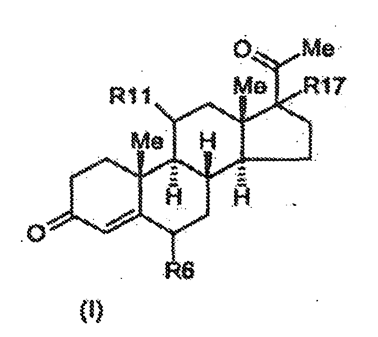

2. The composition according to claim 1, wherein R6 is hydrogen, R11 is hydroxyl and R17 is hydrogen.

3. The composition according to claim 2, wherein the hydroxy derivative of progesterone having formula (I) is 11-α-hydroxyprogesterone, and its pharmaceutically acceptable ester is acetate.

4. The composition according to claim 1, wherein R6 is hydrogen, R11 is hydrogen and R17 is hydroxyl.

5. The composition according to claim 4, wherein the hydroxy derivative of progesterone having formula (I) is 17-α-hydroxyprogesterone, and its pharmaceutically acceptable ester is acetate or hexanoate.

6. The composition according to claim 1, wherein R6 is methyl, R11 is hydrogen and R17 is hydroxyl.

7. The composition according to claim 6, wherein the hydroxy derivative of progesterone having formula (I) is medroxyprogesterone, and its pharmaceutically acceptable ester is acetate.

8. The composition according to any of the above claims, wherein the topical formulation is a lotion that has one or more pharmaceutically acceptable monoalcohols as a dissolvent, and one or more pharmaceutically acceptable polyols as co-dissolvents at a proportion between 20% and 50%, in addition to water at a proportion between 10% and 30%.

9. The composition according to claim 8, wherein the monoalcohol dissolvent is selected from ethanol, isopropanol and its combinations, and the polyol co-dissolvent is selected from propylene glycol, glycerin, sorbitol and its combinations.

10. The composition according to claim 9, wherein the amount of 17-clobetasol propionate is between 0.05% and 0.4% in weight, and the amount of hydroxy derivative of progesterone having formula (I) or its pharmaceutically acceptable ester is between 0.1% and 1.5%.

11. The composition according to claim 10, wherein the amount of 17-clobetasol propionate is between 0.1% and 0.2% in weight, and the amount of hydroxy derivative of progesterone having formula (I) or its pharmaceutically acceptable ester is between 0.3% and 1 %.

12. Use of a combination of 17-clobetasol propionate with a hydroxy derivative of progesterone having formula (I) defined in any of the claims 1 to 7, or with any of its pharmaceutically acceptable esters, for the preparation of a pharmaceutical composition for topical administration for the treatment of inflammatory or allergic skin diseases.

13. Use according to claim 12, wherein the skin disease is psoriasis.

## Patentansprüche

1. Eine pharmazeutische Zusammensetzung zur topischen Verabreichung umfassend eine therapeutisch wirksame Menge von 17-Clobetasol-Propionat, adäquate Mengen von Arzneistoffträgern, die für pharmazeutische oder kosmetische Formulierungen zur topischen Verabreichung verträglich sind, und eine therapeutisch wirksame Menge eines Hydroxyderivats eines Progesterons mit der Formel (I), oder eines pharmazeutisch verträglichen Esters davon, wobei, unabhängig voneinander, R6 aus Wasserstoff oder Methyl ausgewählt ist; R11 aus Wasserstoff oder Hydroxyl ausgewählt ist, und R17 aus Wasserstoff oder Hydroxyl ausgewählt ist, unter der Bedingung, dass R11 und R17 nicht gleichzeitig Wasserstoffe sind.

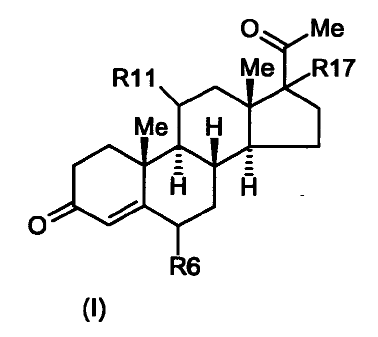

2. Die Zusammensetzung nach Anspruch 1, wobei R6 Wasserstoff ist, R11 Hydroxyl ist und R17 Wasserstoff ist.

3. Die Zusammensetzung nach Anspruch 2, wobei das Hydroxyderivat des Progesterons mit der Formel (I) ein 11-α-Hydroxyprogesteron ist und dessen pharmazeutisch verträglicher Ester Acetat ist.

4. Die Zusammensetzung nach Anspruch 1, wobei R6 Wasserstoff ist, R11 Wasserstoff ist und R17 Hydroxyl ist.

5. Die Zusammensetzung nach Anspruch 4, wobei das Hydroxyderivat des Progesterons mit der Formel (I) ein 17-α-Hydroxyprogesteron ist und dessen pharmazeutisch verträglicher Ester Acetat oder Hexanoat ist.

6. Die Zusammensetzung nach Anspruch 1, wobei R6 Methyl ist, R11 Wasserstoff ist und R17 Hydroxyl ist.

7. Die Zusammensetzung nach Anspruch 6, wobei das Hydroxyderivat des Progesterons mit der Formel (I) ein Medroxyprogesteron ist und dessen pharmazeutisch verträglicher Ester Acetat ist.

8. Die Zusammensetzung nach einem der vorhergehenden Ansprüche, wobei die topische Formulierung eine Lotion ist, die als Lösungsmittel einen oder mehrere pharmazeutisch verträgliche Monoalkohole und als Co-Lösungsmittel einen oder mehrere pharmazeutisch verträgliche Polyole in einem Verhältnis von 20% bis 50% besitzt, und außerdem Wasser in einem Verhältnis von 10% bis 30%.

9. Die Zusammensetzung nach Anspruch 8, wobei das Monoalkohol Lösungsmittel aus Ethanol, Isopropanol und deren Kombinationen ausgewählt ist, und wobei das Polyol Co-Lösungsmittel aus Propylenglykol, Glycerin, Sorbitol und deren Kombinationen ausgewählt ist.

10. Die Zusammensetzung nach Anspruch 9, wobei die Menge des 17-Clobetasol-Propionats zwischen 0.05% und 0.4% Gewicht liegt, und wobei die Menge des Hydroxyderivats eines Progesterons mit der Formel (I) oder eines seiner pharmazeutisch verträglichen Ester zwischen 0.1% und 1.5% Gewicht liegt.

11. Die Zusammensetzung nach Anspruch 10, wobei die Menge des 17-Clobetasol-Propionats zwischen 0.1% und 0.2% Gewicht liegt, und wobei die Menge des Hydroxyderivats eines Progesterons mit der Formel (I) oder eines seiner pharmazeutisch verträglichen Ester zwischen 0.3% und 1 % Gewicht liegt.

12. Verwendung einer Kombination aus 17-Clobetasol-Propionat mit einem Hydroxyderivat eines Progesterons mit der Formel (I) als in den Ansprüchen 1 bis 7 definiert, oder mit einem pharmazeutisch verträglichen Ester davon, für die Herstellung einer pharmazeutischen Zusammensetzung zur topischen Verabreichung für die Behandlung von inflammatorischen oder allergischen Hautkrankheiten.

13. Verwendung nach Anspruch 12, wobei die Hauterkrankung Psoriasis ist.

## Revendications

1. Une composition pharmaceutique pour administration topique qui comprend une quantité thérapeutiquement efficace de 17-propionate de clobétasol, des quantités approprié d'excipients acceptables pour des formulations pharmaceutiques ou cosmétiques pour administration topique, et une quantité thérapeutiquement efficace d'un dérivé hydroxy de progestérone possédant la formule (I), ou de l'un de ses esters pharmaceutiquement acceptables, dans laquelle, indépendamment l'un de l'autre, R6 est choisi entre hydrogène ou méthyle; R11 est choisi entre hydrogène ou hydroxyle; et R17 est choisi entre hydrogène ou hydroxyle, à condition que R11 et R17 ne soient pas l'hydrogène simultanément.

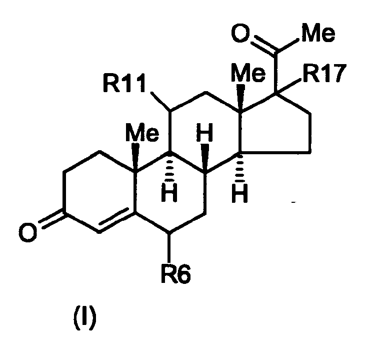

2. La composition selon la revendication 1, dans laquelle R6 est hydrogène, R11 est hydroxyle et R17 est hydrogène.

3. La composition selon la revendication 2, dans laquelle le dérivé hydroxy de progestérone possédant la formule (I) est 11-α-hydroxyprogestérone, et son ester pharmaceutiquement acceptable est l'acétate.

4. La composition selon la revendication 1, dans laquelle R6 est hydrogène, R11 est hydrogène et R17 est hydroxyle.

5. La composition selon la revendication 4, dans laquelle le dérivé hydroxy de progestérone possédant la formule (I) est 17-α-hydroxyprogestérone, et son ester pharmaceutiquement acceptable est l'acétate ou l'hexanoate.

6. La composition selon la revendication 1, dans laquelle R6 est méthyle, R11 est hydrogène et R17 est hydroxyle.

7. La composition selon la revendication 6, dans laquelle le dérivé hydroxy de progestérone possédant la formule (I) est la médroxyprogestérone, et son ester pharmaceutiquement acceptable est l'acétate.

8. La composition selon l'une quelconque des revendications ci-dessus, dans laquelle la formulation topique est une lotion qui possède un ou plusieurs monoalcools pharmaceutiquement acceptables en tant que solvant, et un ou plusieurs polyols pharmaceutiquement acceptables en tant que co-solvants dans une proportion entre 20% et 50%, en plus d'une proportion d'eau entre 10% et 30%.

9. La composition selon la revendication 8, dans laquelle le solvant monoalcool est choisi parmi l'éthanol, l'isopropanol et ses combinaisons, et le co-solvant polyol est choisi parmi le propylène glycol, la glycérine, le sorbitol et ses combinaisons.

10. La composition selon la revendication 9, dans laquelle la quantité de 17-propionate de clobétasol est compris entre 0,05% et 0,4% en poids, et la quantité de dérivé hydroxy de progestérone possédant la formule (I) ou son ester pharmaceutiquement acceptable est compris entre 0,1% et 1,5%.

11. La composition selon la revendication 10, dans laquelle la quantité de 17-propionate de clobétasol est compris entre 0,1% et 0,2% en poids, et la quantité de dérivé hydroxy de progestérone possédant la formule (I) ou son ester pharmaceutiquement acceptable est compris entre 0,3% et 1 %.

12. Utilisation d'une combinaison de 17-propionate de clobétasol avec un dérivé hydroxy de progestérone possédant la formule (I) définie dans l'une quelconque des revendications 1 à 7, ou avec l'une quelconque de ses esters pharmaceutiquement acceptables, pour la préparation d'une composition pharmaceutique pour administration topique pour le traitement de dermopathies inflammatoires ou allergiques.

13. Utilisation selon la revendication 12, où la dermopathie est le psoriasis.
